# EUROPEAN PATENT SPECIFICATION

(11) **EP 1 685 851 B1**
(45) Date of publication and mention of the grant of the patent: **13.05.2009**
(21) Application number: 06101055.9
(22) Date of filing: 31.01.2006
(51) Int. Cl.: A61K 51/12, C12Q 1/04, G01N 33/62

(54) **A method for the diagnosis of helicobacter pylori infection and diagnostic kit for performing the method**
Verfahren zur Diagnose von Heliobacter pylori-Infektion und diagnostisches Kit zur Durchführung des Verfahrens
Procédé de diagnostic d'une infection à l'Helicobacter pylori et kit de diagnostic pour mettre en oeuvre le procédé

(30) Priority: 01.02.2005 EP 05001997; 19.10.2005 US 252886
(43) Date of publication of application: 02.08.2006
(73) Proprietor: Aygen, Sitke, Dr., 51105 Köln (DE)
(72) Inventor: Aygen, Sitke, Dr., 51105 Köln (DE)
(74) Representative: Schreiber, Christoph

(56) References cited:
- EP-A- 0 881 491
- US-A- 6 113 875
- US-A1- 2004 077 965
- MANA F ET AL: "The early effect of proton pump inhibitor therapy on the accuracy of the <13>C-urea breath test" DIGESTIVE AND LIVER DISEASE, W.B. SAUNDERS, vol. 37, no. 1, January 2005 (2005-01), pages 28-32, XP004691289 ISSN: 1590-8658
- DOMINGUEZ-MUNOZ J E: "A CITRIC ACID SOLUTION IS AN OPTIMAL TEST DRINK IN THE 13C-UREA BREATH TEST FOR THE DIAGNOSIS OF HELICOBACTER PYLORI INFECTION" GUT, BRITISH MEDICAL ASSOCIATION, LONDON,, GB, vol. 40, 1997, pages 459-462, XP000949070 ISSN: 0017-5749
- LEODOLTER A ET AL: "CITRIC ACID OR ORANGE JUICE FOR THE 13C-UREA BREATH TEST: THE IMPACT OF PH AND GASTRIC EMPTYING" ALIMENTARY PHARMACOLOGY & THERAPEUTICS, BLACKWELL SCIENTIFIC PUBLICATIONS LTD., CAMBRIDGE, GB, vol. 13, no. 8, August 1999 (1999-08), pages 1057-1062, XP000858021 ISSN: 0269-2813

## Description

### Background of the invention

The present invention relates to a method for the diagnosis of *Helicobacter* pylori infection and a diagnostic kit for performing the method.

There are a number of known methods for diagnosing *Helicobacter pylori* infection, e.g.s the upper digestive tract endoscopy, the stool antigen test (HpSA /Meridian, Milan, Italy), serological method (Pylortest EIA-G III / Orion Diagnostics, Espoo, Finland), antibody detection in urine (Otsuka Diagnostic, Frankfurt, Germany). The Helicobacter breath test INFAI (INFAI GmbH, Cologne, Germany) became the leading method. It is in commercial use with 75mg ¹³C-labeled urea together with up to 1g citric acid for adults and with 45mg ¹³C-labeled urea together with 100ml orange juice for children from the age of 3 years.

US-A-6 113 875 discloses a tablet comprising 50 mg of ¹³C-labeled urea together with 63 mg of citric acid and EP-A-0 881 491 discloses a kit with a 200 ml solution comprising 4.2 g of citric acid and a 50 ml solution comprising 75 mg of ¹³C-labeled urea.

A severe disadvantage of nearly all these tests, however, is that proton pump inhibitors (PPIs) and antacid therapy disturb and give false negative results; see L. Gatta et al, in American Journal of Gastroenterology, 2004 ,p.823 -828.

It therefore is demanded, that these PPIs and antacid therapy must be discontinued 12 to 14 days at least before the ¹³C-urea breath test (UBT) can be performed with reliable results. This increases cost and inconvenience, as the patient must return after at least 12 days. Furtheron the patient suffers from the discontinuation during this time.

### Summary of the invention

It has been one object of the invention to provide a method for the diagnosis of *Helicobacter pylori* for patients taking PPIs or other antacid drugs.

Surprisingly it now was found, that this problem can be solved if the patients taking proton pump inhibitors (PPIs) are administered higher amounts of acids overcompensating for a time of 10 minutes to one hour the activity of the PPI.

It could not be foreseen that such a short-time overcompensation of the PPI-activity would be able to overcome the known long-time negative influence of these drugs on the reliability of the UBT-tests.

There are strong indications that this is also helpful with patients being administered antacid drugs.

It is not only more convenient for the patients to be tested immediately without discontinuation of intake of PPIs or antacid drugs. It is also better accepted to suffer for at most one hour instead of two weeks without the ther-a py.

### Detailed description of the invention

In one embodiment, the invention provides a method for diagnosing a *Helicobacter pylori* infection in a patient treated with proton pump inhibitors (PPIs) comprising the steps of
a) administering to the patient an amount of an acid in the range of 5 to 7 g,
b) collecting a first breath sample or a first blood sample or both from the patient,
c) administering to the patient ¹³C-labeled urea,
d) waiting for a time of 10 to 60 minutes,
e) collecting a second breath sample or a second blood sample or both from the patient,
f) measuring the content of ¹³C in CO₂ of the first and second samples.

The amount of acid is preferably in the range of 5.5 to 6.5 g. Suitable acids are pharmacologically acceptable acids, for example those selected the group of citric acid, malic acid, tartaric acid and mixtures thereof.

An especially useful mixture is a combination of 5 g citric acid, 0.3 g malic acid and 0.2 g tartaric acid.

Preferably the acid is administered in a solution with water, e.g. 150 to 300 ml. Preferably sweeteners are added to get an acceptable taste of the solution. Other components such as flavours or colorants can be added, too.

In one embodiment of the invention, the test is a breath test. In this test, the breath samples are taken prior to administration of the ¹³C-labeled urea and about 30 minutes after administration of the urea. The ¹³C content of CO₂ is then measured from these breath samples. A difference between 00 min value and 30 min value of more than 3.2, preferably more than 4 ppm indicates a *Helicobacter pylori* infection.

In a different embodiment blood samples are selected from the patient prior to administration of the ¹³C-labeled urea and a second blood sample is selected about 15 minutes later. Upon addition of a strong acid, ¹³CO₂ is released from the blood samples and the concentration of ¹³CO₂ can be measured accordingly. The cut-off value for the blood test is 2 ppm, i.e. a difference between 00 min value and 15 min value of more than 2 ppm indicates *Helicobacter pylori* infection.

The measurement of ¹³CO₂ is relative to the content of ¹²CO₂ and ¹³CO₂ together. A suitable method is for example Isotope Ratio Mass-Spectrometry (IRMS).

Suitable amounts of ¹³C-urea depend on the purity of the ¹³C-urea and the type of samples.

¹³C-urea is commercially available in 99% purity. If this ¹³C-urea is used, amounts of 10 to 100 mg are sufficient. If less pure ¹³C-urea is used, the amounts of urea must be correspondingly higher. If, for example the ¹³C content is 50%, about twice the amount is needed.

For the blood test, a sufficient amount is in the range of 5 to 50 mg, preferably about 10 mg. For a breath test, typically the amount is about 75 mg, for children the amount is about 45 mg.

In one embodiment of the invention the treatment with PPI is discontinued for 1, 2 or 3 days, preferably one day, prior to diagnosis of the *Helicobacter pylori* infection. This increases reliability of the method and smaller amounts of acids can be used. A convenient mixture is e.g. 4.5 g citric acid, 0.3 g malic acid and 0.2 g tartaric acid.

In a further embodiment, the acid is administered together with ¹³C-labeled urea. The method comprises the steps of
a) collecting a first breath sample or a first blood sample or both from the patient,
b) administering to the patient an amount of an acid in the range of 5 to 7 g and ¹³C-labeled urea
c) waiting for a time of 10 to 60 minutes,
d) collecting a second breath sample or a second blood sample or both from the patient,
e) measuring the content of ¹³C in CO₂ of the first and second samples.

The acids are administered preferably in form of aqueous solutions.

Such solutions can be prepared from solid or liquid acids immediately before the test. They also can be prepared in larger amounts and can be stored.

The breath test is run in the usual and well established method described in the literature and the patient instruction sheets, which are available also to the medical doctor. The samples are analysed for example by gas isotope ratio mass spectroscopy or infrared spectrometer.

The following examples compare the results of the test according to the present invention with 5 g citric acid, 0.3 g malic acid and 0.2 tartaric acid (New ¹³C UBT) with the tests according to the prior art (¹³C -UBT Standard with 1g citric acid).

152 consecutive Helicobacter pylori positive patients have been studied.

Upper endoscopy was performed in each patient. During endoscopy, multiple biopsies were taken from antrum and corpus. Histology, RUT (Rapid Urease Test), and culture were used to assess the *Helicobacter pylori* status, Histology was performed using the H&E, and the modified Giemsa stain.

Histology was scored using the Up date Sydney System. Patients were considered infected if 2 out of 3 tests were positive or if culture was positive alone.

Patients found to be positive were randomised using a randomisation list to perform a standard ¹³C-UBT or the new ¹³C-UBT formulation.

After UBT they received a standard dose of esomeprazole (40 mg/day) to be taken in the morning 30 min before breakfast.

Patients were asked to return at day 14^{th} and 28^{th} of treatment with esomeprazole to perform the UBTs and to check the compliance with medication.

Patients were also asked to return at day 7^{th} and 14^{th} after stopping treatment with esomeprazole to perform the UBTs.

Sensitivity, difference between proportions for independent samples and their Cis (Confidence intervals) were calculated according to Wilson and Newcombe method's.

Sensitivity was assessed according to ITT (Intension to Treat) Analysis i.e. patients dropped were considered as False Negative results.

The p value was calculated using Fischer's exact test. NNT (Number Needed to Treat) was also calculated.

Multiple logistic regression for the independent determinants of sensitivity was performed using the following variables:
Type of UBT
Sex
Age
Body Mass Index
Score for *Helicobacter pylori* in Antrum (density)
Score for *Helicobacter pylori* in Corpus (density)

Multiple logistic regression was performed according to ITT Analysis i.e. patients dropped were considered as False Negative results.

From 152 *Helicobacter pylori* positive patients randomised 76 patients were tested with the method of the invention and 76 patients were tested with the formulation of the prior art.

### The results after 14^{th} day of PPI

The test according to the invention gave 71 True Positive, 3 False Negative, 2 Drop-Out.

The standard gave 59 True Positive, 14 False Negative , and 3 Drop-Out.

The sensitivity increased from 75,7% to 95,8% with a p value of 0.0005.

### The results after 28^{th} day of PPI

Method of the invention 67 True Positive, 7 False Negative, 2 Drop-Out

Method of prior art 43 True Positive, 30 False Negative, 3 Drop-Out.

### The results after 7^{th} day of wash-out

New Test: 74 True Positive, 2 Dropout

Standard Test: 73 True Positive 3 Drop-Out

### The results after 14^{th} day of wash-out

New Test: 74 True Positive, 2 Drop-Out

Standard Test: 73 True Positive, 3 Drop-Out.

These results clearly show the highly significant improvement with the new formulation and confirm the known demand of at least 12 day discontinuation of PPI treatment in conventional test. This now can be avoided with the method according to the present invention.

## Claims

1. A method for diagnosing a *Helicobacter pylori* infection in a patient treated with proton-pump-inhibitors (PPIs) comprising the steps of
I. a) administering to the patient an amount of an acid in the range of 5 to 7 g,
I. b) collecting a first breath sample or a first blood sample or both from the patient,
I. c) administering to the patient ¹³C-labeled urea,
I. d) waiting for a time of 10 to 60 minutes,
I. e) collecting a second breath sample or a second blood sample or both from the patient,
I. f) measuring the content of ¹³C in the CO₂ of the first and second samples
or
II. a) collecting a first breath sample or a first blood sample or both from the patient,
II. b) administering to the patient an amount of an acid in the range of 5 to 7 g and ¹³C-labeled urea
II. c) waiting for a time of 10 to 60 minutes,
II. d) collecting a second breath sample or a second blood sample or both from the patient,
II. e) measuring the content of ¹³C in the CO₂ of the first and second samples. ,

2. The method of to claim 1 wherein the amount of acid is in the range of 5.5 to 6.5 g.

3. The method of claim 1 or 2 wherein the acid is selected from the group of citric acid, malic acid, tartaric acid and mixtures thereof.

4. The method of claim 1 or 2 wherein the acid is citric acid, alone or a mixture from citric acid, malic acid and tartaric acid.

5. The method of any one of claims 1 to 4 wherein the acid is administered as a solution with water, optional with compounds selected from the group of sweeteners, flavours and colorants.

6. The method of any one of claims 1 to 5 wherein the time of step I. d) or II. c) is in the range of 10 to 15 minutes and the first and the second samples are blood samples.

7. The method of any one of claims 1 to 6 wherein the dme of step I. d) or II. c) is in the range of 20 to 40 minutes and the first and second samples are breath samples.

8. The method of any one of claims 1 to 7 wherein the patient discontinues treatment with PPI for 1 to 3 days prior to diagnosis of *Helicobacter pylori* infection.

9. The method of any one of claims 1 to 8, wherein the amount of ¹³C-labelled urea corresponds to 10 to 100 mg 99% ¹³C-urea.

10. A kit for diagnosis of *Helicobacter pylori* comprising
a) either an acidic aqueous solution containing 5 to 7 g of an acid and an amount of ¹³C-labelled urea corresponding to 10 to 100 mg 99% ¹³C-urea, or
b) a solid acid powder pack comprising 5 to 7 g of an acid and a separate urea pack comprising an amount of ¹³C-labelled urea corresponding to 10 to 100 mg 99% ¹³C-urea
c) sample container for blood or breath collection
d) a patient instruction sheet

11. The kit of claim 10 wherein the amount of acid is in the range of 5.5 to 6.5 g.

## Patentansprüche

1. Verfahren zum Diagnostizieren einer *Helicobacter-pylori-*Infektion bei einem Patienten, der mit Protonenpumpenhemmern (PPI) behandelt wird, umfassend die folgenden Schritte:
I.a) Verabreichen einer Menge einer Säure im Bereich von 5 bis 7 g an den Patienten;
I.b) Nehmen einer ersten Atemprobe oder einer ersten Blutprobe oder beides bei dem Patienten;
I.c) Verabreichen von ¹³C-markiertem Harnstoff an den Patienten;
I.d) Warten während eines Zeitraums von 10 bis 60 Minuten;
I.e) Nehmen einer zweiten Atemprobe oder einer zweiten Blutprobe oder beides bei dem Patienten;
I.f) Messen des Gehalts an ¹³C im CO₂ der ersten und der zweiten Proben;
oder
II.a) Nehmen einer ersten Atemprobe oder einer ersten Blutprobe oder beides bei dem Patienten;
II.b) Verabreichen einer Menge einer Säure im Bereich von 5 bis 7 g und von ¹³C-markiertem Harnstoff an den Patienten;
II.c) Warten während eines Zeitraums von 10 bis 60 Minuten;
II.d) Nehmen einer zweiten Atemprobe oder einer zweiten Blutprobe oder beides bei dem Patienten;
II.e) Messen des Gehalts an ¹³C im CO₂ der ersten und der zweiten Proben.

2. Verfahren gemäß Anspruch 1, wobei die Menge der Säure im Bereich von 5,5 bis 6,5 g liegt.

3. Verfahren gemäß Anspruch 1 oder 2, wobei die Säure aus der Gruppe Zitronensäure, Äpfelsäure, Weinsäure und Gemischen davon ausgewählt ist.

4. Verfahren gemäß Anspruch 1 oder 2, wobei es sich bei der Säure um Zitronensäure allein oder ein Gemisch von Zitronensäure, Äpfelsäure und Weinsäure handelt.

5. Verfahren gemäß einem der Ansprüche 1 bis 4, wobei die Säure als Lösung mit Wasser verabreicht wird, gegebenenfalls mit Verbindungen, die aus der Gruppe Süßungsmittel, Aromen und Farbstoffe ausgewählt sind.

6. Verfahren gemäß einem der Ansprüche 1 bis 5, wobei der Zeitraum von Schritt I.d) oder II.c) im Bereich von 10 bis 15 Minuten liegt und die erste und die zweite Probe Blutproben sind.

7. Verfahren gemäß einem der Ansprüche 1 bis 6, wobei der Zeitraum von Schritt I.d) oder II.c) im Bereich von 20 bis 40 Minuten liegt und die erste und die zweite Probe Atemproben sind.

8. Verfahren gemäß einem der Ansprüche 1 bis 7, wobei der Patient die Behandlung mit PPI vor der Diagnose der *Helicobacter-pylori*-Infektion 1 bis 3 Tage lang unterbricht.

9. Verfahren gemäß einem der Ansprüche 1 bis 8, wobei die Menge des ¹³C-markierten Harnstoffs 10 bis 100 mg 99%-igem ¹³C-Harnstoff entspricht.

10. Kit für die Diagnose von *Helicobacter pylori,* umfassend:
a) entweder eine saure wässrige Lösung, die 5 bis 7 g einer Säure sowie eine Menge ¹³C-markierten Harnstoffs, die 10 bis 100 mg 99%-igem ¹³C-Harnstoff entspricht, enthält; oder
b) eine Packung mit festem saurem Pulver, die 5 bis 7 g einer Säure umfasst, und eine getrennte Harnstoffpackung, die eine Menge ¹³C-markierten Harnstoffs, die 10 bis 100 mg 99%-igem ¹³C-Harnstoff entspricht, enthält;
c) einen Probenbehälter zum Auffangen von Blut oder Atem;
d) ein Blatt mit Anweisungen für den Patienten.

11. Kit gemäß Anspruch 10, wobei die Menge der Säure im Bereich von 5,5 bis 6,5 g liegt.

## Revendications

1. Procédé de diagnostic d'une infection à *Helicobacter pylori* chez un patient traité avec des inhibiteurs de la pompe à protons (IPP), comprenant les étapes consistant à :
I.a) administrer au patient une quantité d'un acide dans la gamme de 5 à 7 g,
I.b) recueillir un premier échantillon d'haleine ou un premier échantillon de sang ou les deux chez le patient,
I.c) administrer au patient de l'urée marquée au ¹³C,
I.d) attendre pendant une durée allant de 10 à 60 minutes,
I.e) recueillir un deuxième échantillon d'haleine ou un deuxième échantillon de sang ou les deux chez le patient,
I.f) mesurer la concentration de ¹³C dans le CO₂ des premiers et deuxièmes échantillons,
ou
II.a) recueillir un premier échantillon d'haleine ou un premier échantillon de sang ou les deux chez le patient,
II.b) administrer au patient une quantité d'un acide dans la gamme de 5 à 7 g et de l'urée marquée au ¹³C,
II.c) attendre pendant une durée allant de 10 à 60 minutes,
II.d) recueillir un deuxième échantillon d'haleine ou un deuxième échantillon de sang ou les deux chez le patient,
II.e) mesurer la concentration de ¹³C dans le CO₂ des premiers et deuxièmes échantillons.

2. Procédé selon la revendication 1, dans lequel la quantité d'acide est dans la gamme de 5,5 à 6,5 g.

3. Procédé selon la revendication 1 ou 2, dans lequel l'acide est choisi dans le groupe comprenant l'acide citrique, l'acide malique, l'acide tartrique et les mélanges de ceux-ci.

4. Procédé selon la revendication 1 ou 2, dans lequel l'acide est l'acide citrique seul ou un mélange d'acide citrique, d'acide malique ou d'acide tartrique.

5. Procédé selon l'une quelconque des revendications 1 à 4, dans lequel l'acide est administré sous la forme d'une solution aqueuse, éventuellement avec des composés choisis dans le groupe des édulcorants, des arômes et des colorants.

6. Procédé selon l'une quelconque des revendications 1 à 5, dans lequel la durée de l'étape I.d) ou II.c) est dans la gamme de 10 à 15 minutes et les premier et deuxième échantillons sont des échantillons de sang.

7. Procédé selon l'une quelconque des revendications 1 à 6, dans lequel la durée de l'étape I.d) ou II.c) est dans la gamme de 20 à 40 minutes et les premier et deuxième échantillons sont des échantillons d'haleine.

8. Procédé selon l'une quelconque des revendications 1 à 7, dans lequel le patient arrête le traitement aux IPP pendant 1 à 3 jours avant le diagnostic de l'infection à *Helicobacter pylori.*

9. Procédé selon l'une quelconque des revendications 1 à 8, dans lequel la quantité d'urée marquée au ¹³C correspond à 10 à 100 mg de ¹³C-urée à 99%.

10. Trousse de diagnostic d'*Helicobacter pylori* comprenant :
a) soit une solution aqueuse acide contenant 5 à 7 g d'un acide et une quantité d'urée marquée au ¹³C correspondant à 10 à 100 mg de ¹³C-urée à 99%,
b) soit un sachet de poudre acide compacte comprenant 5 à 7 g d'un acide et un sachet d'urée séparé comprenant une quantité d'urée marquée au ¹³C correspondant à 10 à 100 mg de ¹³C-urée à 99%,
c) un récipient à échantillons pour la collecte d'échantillons de sang ou d'haleine,
d) une notice d'instructions destinée au patient.

11. Trousse selon la revendication 10, dans laquelle la quantité d'acide est dans la gamme de 5,5 à 6,5 g.
